(19) Europäisches Patentamt
European Patent Office
Office européen des brevets

(11) Publication number: **0 264 457 B1**

# (12) EUROPEAN PATENT SPECIFICATION

(45) Date of publication of patent specification: **31.03.93** (51) Int. Cl.[5]: **C12P 41/00**

(21) Application number: **87902734.0**

(22) Date of filing: **16.04.87**

(86) International application number:
**PCT/JP87/00244**

(87) International publication number:
**WO 87/06269 (22.10.87 87/23)**

Divisional application 92200537.6 filed on
16/04/87.

## (54) PROCESS FOR PREPARING OPTICALLY ACTIVE CYCLOPROPANECARBOXYLIC ACIDS.

(30) Priority: **16.04.86 JP 89803/86**
**24.04.86 JP 95444/86**

(43) Date of publication of application:
**27.04.88 Bulletin 88/17**

(45) Publication of the grant of the patent:
**31.03.93 Bulletin 93/13**

(84) Designated Contracting States:
**CH DE FR GB LI NL**

(56) References cited:
**EP-A- 0 164 573**
**JP-A-60 199 393**
**JP-A-60 244 294**

**CHEMICAL ABSTRACTS, vol. 102, no. 23, June 1985, page 490, abstract no. 202608q, Columbus, Ohio, US; JP-A-59 210 892 (SUMITOMO CHEMICAL CO., LTD) 29-11-1984**

(73) Proprietor: **SUMITOMO CHEMICAL COMPANY, LIMITED**
**Kitahama 4-chome 5-33**
**Chuo-ku Osaka 541(JP)**

(72) Inventor: **NISHIZAWA, Kanji**
**Sumika-Ashiya Apt. 203 15-10, Kusunokicho**
**Ashiya-shi Hyogo 659(JP)**
Inventor: **MITSUDA, Satoshi**
**6-34, Izumigaoka**
**Takarazuka-shi Hyogo 665(JP)**
Inventor: **KOMAKI, Ryohei**
**Sumika-Mefuryo 2-14-7, Mefu**
**Takarazuka-shi Hyogo 665(JP)**
Inventor: **SUGIMOTO, Masako**
**Santoso-Mansion 205 2-7, Yumotocho**
**Takarazuka-shi Hyogo 665(JP)**
Inventor: **SUGIKI, Chiaki**
**2-5-J-1004, Tenno**
**Ibaraki-shi Osaka 567(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 264 457 B1

**CHEMICAL ABSTRACTS, vol. 104, no. 21, May 1986, page 514, abstract no. 184884m, Columbus, Ohio, US; && JP-A-60 199 393 (SUMITOMO CHEMICAL CO., LTD) 08-10-1985**

Inventor: **OGAMI, Yasutaka**
**Famille Mikage 116-11, Mikagecho-Gunge-Babazoe**
**Higashinada-ku, Kobe-shi Hyogo 658(JP)**
Inventor: **SONODA, Kazumi**
**6-37, Zenpojicho**
**Amagasaki-shi Hyogo 661(JP)**
Inventor: **KISHIMOTO, Fumitaka**
**4-8-1, Daiwahigashi**
**Kawanishi-shi Hyogo 666-01(JP)**

(74) Representative: **Myerscough, Philip Boyd et al**
**J.A.Kemp & Co. 14, South Square Gray's Inn**
**London, WC1R 5EU (GB)**

## Description

The present invention relates to a method for producing an optically active (+)-cis cyclopropane carboxylic acid or salt thereof with microorganisms or esterases produced by the microorganisms.

The cyclopropane carboxylic acids of interest constitute the acid moieties of low toxic and rapidly acting insecticidal esters generally termed pyrethroids such as allethrin, permethrin, decamethrin and teffuluthrin.

The cyclopropane carboxylic acids contain two asymmetric carbon atoms at the 1-position and the 3-position and therefore have four diastereomers. Of these isomers, those having the absolute configurations of "1R, 3S" and "1R and 3R" are termed "(+)-cis isomer" and "(+)-trans isomer" (according to RS nomenclature), respectively, because the optical rotation of these isomers is (+) in a specific solvent and the substituent groups are in the cis-form and transform, respectively. The other two isomers having the absolute configurations of "1S, 3S" and 1S, 3R" are termed "(-)-cis isomer" and "(-)-trans isomer", respectively, because the optical rotation of these isomers is (-) in a specific solvent and the substituent groups of them are in the cis-form and trans-form, respectively. Only the (+)-isomers have insecticidal activities as pyrethroid esters and the (-)-isomers have almost no insecticidal activities as pyrethroids.

The relative effects of the cis and the trans isomers vary according to the kinds of harmful insects to be killed and the type of effect.

It is possible to use the (+)-trans-pyrethroidal compound and (+)-cis- compound independently for different purposes. Accordingly, the production of (+)-cyclopropane carboxylic acids in an effective manner is industrially important.

The presently known major method for the production of (+)-isomers is organosynthetic optical resolution, but development of more economical methods for optical resolution is now desired for production of (+)-isomers because the organosynthetic optical resolution requires a relatively expensive optically active reagent or a complicated step. It is known to produce optically active (+)-trans acid by resolving cyclopropane carboxylates by asymmetric hydrolysis with pig liver esterase (e.g. Schneider et al., Angewande Chemie International Edition in English 23. 64 (1984) or with a microbial esterase (JP-A-244295/1985).

However, the former method is industrially disadvantageous because of the expense and only limited supply of pig liver esterase. As for the latter method, it is reported that the trans-isomer of cyclopropane carboxylic acid ester is preferentially resolved by asymmetric hydrolysis but the method can not be industrially applied because the yield of (+)-trans-cyclopropane carboxylic acid is as low as 31 mg/100ml culture medium, and the concentration of the substrate and the optical purity of the resulting (+)-trans cyclopropane carboxylic acid are low.

This invention seeks to provide an industrially advantageous method for producing (+)-cis-cyclopropane carboxylic acids or salts. According to the invention there is provided a process for producing an optically active (+)-cis-cyclopropane carboxylic acid or salt thereof which comprises reacting a (±)-cis-cyclopropane carboxylic acid ester represented by the formula (II)

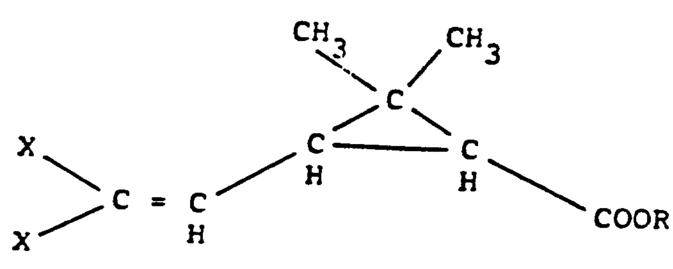

(II)

wherein each X, which may be the same or different represents chlorine, bromine, methyl or trifluoromethyl, and R represents a $C_1$-$C_4$ alkyl group or a $C_1$-$C_4$ alkyl group substituted by halogen atom, which formula does not indicate the configuration of the ester, with a microorganism selected from

| | |
|---|---|
| Rhodosporidium toruloides | IFO-0559 |
| Rhodosporidium toruloides | IFO-0871 |
| Rhodosporidium toruloides | IFO-8766 |
| Rhodotorula glutinis | IFO-1501 |
| Candida tropicalis | IFO-0618 |
| Hansenula anomala var. ciferii | IFO-0994 |

| | |
|---|---|
| Torulopsis candida | IFO-0768 |
| Arthrobacter citreus | IFO-12957 |
| Pseudomonas putida | IFO-12996 |
| Escherichia coli | IFO-13168 |
| Bacillus licheniformis | IFO-12195 |
| Flavobacterium capsulatum | IFO-12533 |
| Chromobacterium chocolatum | IFO-3758 |
| Achromobacter lyticus | ATTC-21456 |
| Rhizomucor pusillus | IFO-9856 |
| Flammulina velutipes | IFO-7046 |
| Geotrichum candidum | IFO-4597 |
| Dipodscus uninucleatus | ATTC-14626 |
| Beauveria bassiana | ATTC-26037 |
| Metschinikowia pulcherrima | IFO-0561 |
| Kluyveromyces lactis | IFO-1090 |
| Frateuria aurantia | IFO-3247 |
| Klebsiella pneumoniae | IFO-12059 and |
| Pediococcus acidilactici | IFO-3076 |

or an esterase produced by said microorganism to selectively and asymmetrically hydrolyse the ester of formula (II) to produce an optically active (+)-cis-cyclopropane carboxylic acid of formula (I) or salt thereof:

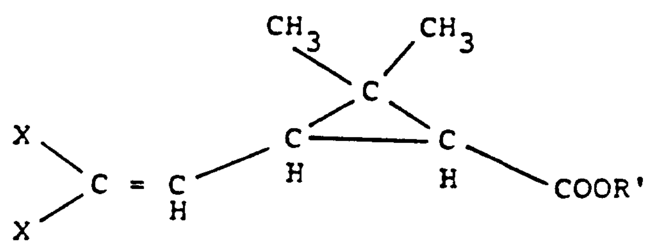

(I)

wherein X is as herein defined and R' represents hydrogen or a metal ion, which formula does not indicate the configuration of the acid, and (-)-cis-cyclopropane carboxylic acid ester of formula (II) and then recovering the optically active (+)-cis-cyclopropane carboxylic acid of formula (I) or salt thereof. The resulting (+)-cis-cyclopropane carboxylic acids or their salts can be recovered with high optical purity.

In a preferred embodiment the invention provides a process for producing a (+)-cis-cyclopropane carboxylic acid or salt thereof represented by the formula (I-a)

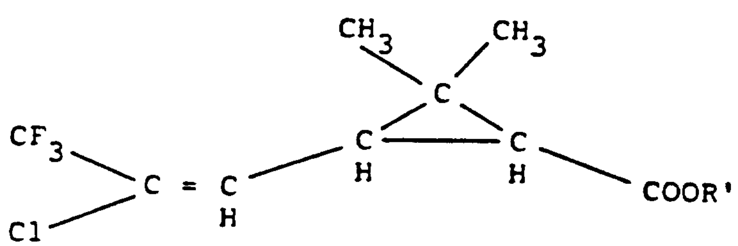

(I-a)

wherein R' represents hydrogen atom or metal ion, which formula does not indicate the configuration of the derivative or salt, which process comprises reacting a (±)-cis-cyclopropane carboxylic acid ester of the formula a(II-a):

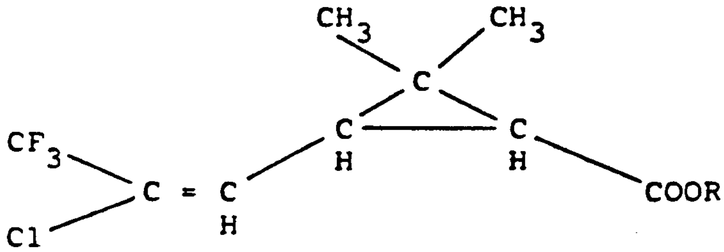

(II-a)

wherein R represents a $C_1$-$C_4$ alkyl group or $C_1$-$C_4$ alkyl group substituted by halogen atom, which formula does not indicate the configuration, with a microorganism which is Arthrobacter globiformis IFO-12958 or Bacillus sp. DC-1 (BP-1254) or an esterase produced by said microorganism.

The esters represented by the formula (II), which are starting materials in this invention, are easily obtained by well-known methods, for example, Pestic. Sci. Vol. 11, pp 156-164 (1980). As the esters used as the starting materials, methyl ester, ethyl ester, propyl ester, butyl ester, monochloroethyl ester, monochloropropyl ester or monobromoethyl ester, are conveniently used. Especially, methyl ester, ethyl ester, and monochloroethyl ester are advantageous because of commercial availability and ease in handling.

Example of salts of cyclopropane carboxylic acids represented by the formula (I) is alkali metal salt such as sodium salt.

Cultivation of the microorganisms is carried out in the usual procedure. For example, microorganisms are cultured in a liquid medium, for instance, inoculating microorganisms in a sterilized liquid medium and then cultivating usually at 20-40 °C, for 1-8 days with shaking. Alternatively, a solid medium may be used.

Any composition of the culture medium may be used, as long as the medium is familiar to cultivation of microorganisms and is utilizable by the microorganism used in this invention. As carbon sources and nitrogen sources in the medium, for example, glucose, starch, dextrin, molasses, fats and fatty oils, soybean powder, defatted soybean powder, fatty bean cake and corn steep liquor are employed. Ammonium sulfate, dipotassium hydrogenphosphate, magnesium sulfate or urea may, for example, be used as inorganic salts in the medium. The cyclopropane carboxylic acid esters represented by the formula (II) or a fatty acid ester may be added to the medium.

In the method of this invention, asymmetric hydrolysis of cyclopropane carboxylic acid esters represented by the formula (II) is performed by mixing the esters (II) with a culture solution of the above microorganisms, a cell suspension, an esterase-containing aqueous solution such as a liquid esterase extract or its concentrated solution or their processed products, such as crude esterase or purified esterase, and then stirring or shaking the thus prepared mixture.

It is advantageous to perform the reaction at 10-65 °C. Since the stability of the esterase is likely to decrease at high temperature and the reaction rate is small at low temperatures, it is preferable that the reaction temperature be within the range of 20-50 °C. It is desirable that the pH of the mixture during reaction be 3-11, preferably around 5-10. It is preferable that buffers, such as phosphate buffer, be used to keep suitable pH during reaction.

Reaction time, which varies depending on the reaction conditions such as amount of esterase produced by the microorganisms or temperature during reaction, is within the range of 2-3 to about 150 hours.

It is advantageous that the concentration of cyclopropane carboxylic acid esters represented by the formula (II) as a substrate when asymmetric hydrolysis is effected be 0.1-50 wt%, preferably 1-25 wt% based on a reaction liquid.

To the reaction mixture, 0.01-1 % of surface active agents such as Triton X-100, Tween 80 or Brij 58 may be added, if necessary.

Cells of the microorganisms or the esterases may be used in the immobilized form, which is prepared by immobilizing them in a usual manner on inorganic or organic carriers, for example, zeolite, alumina, polysaccharide, polyamide, polystyrene resin, polyacrylic resih, and polyurethane resin.

Cell suspension, suspension of ground cells or aqueous solution containing esterase, arc prepared according to the usual method. Cell suspension is prepared by separating the cells harvested from culture medium by centrifugation or ultra filtration and suspending the cells in distilled water, ion-exchanged water, or buffer solution containing inorganic or organic salts, such as phosphate buffer solution. Suspension of ground cells is prepared by applying ultrasonic treatment, high pressure-breaking treatment by Manton-Gaulinhomogenizer or French Press or lytic enzyme treatment to the cell suspension. If necessary, the suspension may be changed to crude esterase solution by removing ground cell residue from the above

suspension using centrifugation or ultrafiltration. Esterase is obtained from the suspension of ground cells by a conventional method, such as salting out with ammonium sulfate or sodium sulfate, or precipitation with organic solvents using hydrophylic organic solvents such as ethanol, propylalcohol or acetone. Aqueous solution containing esterase is prepared by dissolving this obtained esterase in distilled water, ion-exchanged water, or buffer containing inorganic or organic salts, for example, phosphate buffer solution.

After the asymmetric hydrolysis is over, the liberated optically active cyclopropane carboxylic acid derivative represented by the formula (I) is separated from the unaltered ester and recovered by, for example, extraction with a solvent, column chromatography or fractional distillation. For example, the reaction mixture is subjected to extraction with an organic solvent such as methyl isobutyl ketone, chloroform, ether, benzene or toluene and then the extract is subjected to fractional distillation under reduced pressure to separate the liberated optically active cyclopropane carboxylic acid derivatives represented by the formula (I) from the unaltered esters.

One of microorganisms utilized in this invention is Bacillus sp. DC-1, which is a novel microorganism the characteristics of which are as follows:-

(a) Morphological characteristics

1) From and size of cell:
Rod, (0.5 - 0.6) µm x (1.2 - 1.7) µm. Occuring singly or in short chains.
2) Polymorphism: None
3) Motility: Motile by peritrichous flagella.
4) Spore formation: Endospores formed. Spherical or slightly oval with 0.4 - 0.6 µm in diameter. Spores formed in a terminal position of the vegetative cell, having swell.
5) Gram staining: Negative
6) Acid fastness: Negative

(b) Cultural characteristics on various mediums

1) Bouillon agar plate (35° C, 24 hours)
Shape: Circular and projected form
Margine: None
Surface: Smooth and lustrous
Color tone: Translucent and yellowish white
2) Bouillon agar slant (35° C, 24 hours)
Growth degree: Moderate, growing like spread cloth or beads-like
Surface: Smooth and lustrous
Color tone: Translucent and yellowish white
3) Bouillon liquid (35° C, 24 hours)
Growth degree: Moderate
Coloring / discoloring: None
Pellicle: Not formed
Sediment: Formed
4) Bouillon gelatine stab (35° C, 14 days)
No liquefaction.
5) Litmus milk (35° C, 14 days)
Slightly alkaline. No coagulation nor peptonization.

(c) Physiological characteristics:
Cultured at 35° C for 1 - 5 days. Negative ones were observed up to 14 days.

1) Reduction of nitrate: Positive Nitrite produced from nitrate.
2) Denitrification: Negative
3) MR test: Negative
4) VP test: Negative
5) Production of indole: Negative
6) Production of hydrogen sulfide: Negative
7) Hydrolysis of starch: Negative
8) Utilization of citric acid:
Koser medium: Negative
Christensen medium: Positive
9) Utilization of inorganic nitrogen sources:
Stanier et al's medium modified by Yamazato et al: (Yamazato et al. J. Gen. Appl. Microbiol. (1982) 28:195-213)
Sodium succinate was used as the sole carbon source.

Nitrate: Not utilized
Ammonium salt: Utilized.
10) Pigmentation: Negative
11) Urease:
Christensen urea medium: Positive
12) Oxidase: Positive
13) Catalase: Positive
14) Range of growth:
Growth temperature: 10-45 °C (optimum 30-35 °C)
Growth pH: 6.0-9.5 (optimum 8.5-9.0)
15) Anaerobic or aerobic growth: Aerobic
16) OF test: Negative
17) Production of acid or gas from saccharides:

| | | Acid | Gas |
|---|---|---|---|
| (1) | L-arabinose | - | - |
| (2) | D-xylose | - | - |
| (3) | D-glucose | - | - |
| (4) | D-mannose | - | - |
| (5) | D-fructose | - | - |
| (6) | D-galactose | - | - |
| (7) | Maltose | - | - |
| (8) | Sucrose | - | - |
| (9) | Lactose | - | - |
| (10) | Trehalose | - | - |
| (11) | D-sorbitol | - | - |
| (12) | D-mannitol | - | - |
| (13) | Inositol | - | - |
| (14) | Glycerin | - | - |
| (15) | Starch | - | - |

Reference is made to Bergey's Manual of Determinative Bacteriology, 8th Ed (1974). The strain is identified as that belonging to genus Bacillus, since it is able to grow under aerobic conditions and forms endospores. The present strain is close to but different from Bacillus sphaericus and Bacillus pasteurii, as shown in the table below:

| | the Present strain | Bacillus sphaericus | Bacillus pasteurii |
|---|---|---|---|
| Reduction of nitrate | + | - | - |
| Requirement of urea or ammonia | - | - | - |

In view of the above facts, the present inventors have recognized the strain to be a novel one belonging to genus Bacillus and nominated it Bacillus sp. DC-1. The strain was deposited with Fermentation Research Institute, Industrial Technology Agency, Japan (Address: 1-3, Higashi 1-chome, Yatabe-cho, Tukuba-Gun, Ibaragi, JAPAN) under FERM BP-1254. The strain was first deposited as FERM P-8719 on March 31, 1986, then renumbered as FERM BP-1254 under Budapest Treaty on January 12, 1987.

This invention will be explained more precisely according to the following examples.

Examples 1 - 23

First, 100 ml each of the following liquid media was put in a 500 ml flask and sterilized: a malt-extract-yeast-extract medium (pH 6.5, prepared by dissolving 5.0 g of peptone, 10.0 g of glucose, 3.0 g of malt extract and 3.0 g of yeast extract in 1ℓ of water) for yeasts and fungi (examples 1-7 and 15-21,); a sugar broth medium (pH 7.2, prepared by dissolving 10.0 g of glucose, 5.0 g of peptone, 5.0 g of meat extract and 3.0 g of sodium chloride in 1ℓ of water) for bacteria and actinomycetes (examples 8-14, and 22-23). A

loopful of cells of each microorganism shown in Table 1 growing on slant culture was inoculated in the medium and the mixture was subjected to shaking culture at 30° C for 20 hours.

1.0 g of ethyl (±)-cis-2,2-dimethyl-3-(2,2-dichlorovinyl)cyclopropane carboxylate of formula (II), in which X represents chlorine atom and R represents ethyl group, was added to the medium above and the mixture was allowed to react with shaking at 30° C for 40 hours. After the reaction was over, the pH of the reaction mixture was adjusted to 2 or lower with concentrated hydrochloric acid and the mixture was subjected to extraction with methyl isobutyl ketone. Next, an internal standard (dimethyl phthalate) was added to the extract and the mixture was analyzed by gas chromatography (column: 3% Thermon 3000, 1.1 m, 140° C). Then, the yield of 2,2-dimethyl-3-(2,2-dichlorovinyl)cyclopropane carboxylic acid [in formula (I), X is Cl and R is H] and the recovery rate of the ethyl 2,2-dimethyl-3-(2,2-dichlorovinyl)cyclopropane carboxylate used as the starting material were calculated by comparing their peak areas with that of dimethyl phthalate. It was found that all of the remaining ethyl 2,2-dimethyl-3-(2,2-dichlorovinyl)cyclopropane carboxylate which was not converted to 2,2-dimethyl-3-(2,2-dichlorovinyl)cyclopropane carboxylic acid was recovered. After that, the extract was mixed with IN sodium hydroxide solution to extract 2,2-dimethyl-3-(2,2-dichlorovinyl)-cyclopropane carboxylic acid as sodium salt into the aqueous layer. Next, the pH of the aqueous layer was adjusted to 2 or lower with HCl solution to liberate 2,2-dimethyl-3-(2,2-dichlorovinyl)cyclopropane carboxylic acid which was then extracted by using methyl isobutyl ketone. After that, the extract was concentrated and evaporated to dryness to obtain substantially pure 2,2-dimethyl-3-(2,2-dichlorovinyl)cyclopropane carboxylic acid.

After 10 mg of thus produced 2,2-dimethyl-3-(2,2-dichlorovinyl)cyclopropanecarboxylic acid was dissolved in 1 ml of toluene, equal molar amount each of thionyl chloride, pyridine and (+)-2-octanol were added and the mixture was allowed to react to produce diastereomers of ester of (+)-2-octanol and 2,2-dimethyl-3-(2,2-dichlorovinyl)cyclopropane carboxylic acid. Optical isomers assay in the diastereomers was performed by gas chromatography (column: 10% DCQF-1, 5.1 m, 180° C).

The results are shown in the Table 1 in which the yield of 2,2-dimethyl-3-(2,2-dichlorovinyl)-cyclopropane carboxylic acid represents the molar yield of 2,2-dimethyl-3-(2,2-dichlorovinyl)cyclopropane carboxylic acid relative to ethyl (+)-cis-2,2-dimethyl-3-(2,2-dichlorovinyl)cyclopropane carboxylate contained in ethyl (±)-cis-2,2-dimethyl-3-(2,2-dichlorovinyl)cyclopropane carboxylate used as the starting material.

Examples 24 - 27

The same reaction and analysis as used in Examples 1-23 were performed except that 1.0 g of ethyl (±)-cis-2,2-dimethyl-3-(2,2-dimethylvinyl)cyclopropane carboxylate represented by the formula (II) wherein X represents methyl group and R represents ethyl group, was used as a substrate.

As a result, (+)-cis-2,2-dimethyl-3-(2,2-dimethylvinyl)cyclopropane carboxylic acid represented by the formula (I) wherein X represents methyl group and R represents hydrogen atom, was obtained. The results are shown in the Table 2.

Examples 28 - 31

The same reaction and analysis as used in Example 1-23 were performed except that 1.0 g of methyl (±)-cis-2,2-dimethyl-3-(2,2-dibromovinyl)cyclopropane carboxylate represented by the formula (II) wherein X represents bromine atom and R represents methyl group, was used as a substrate to obtain (+)-cis-2,2-dimethyl-3-(2,2-dibromovinyl)cyclopropane carboxylic acid [in the formula (I), X is bromine atom and R is hydrogen atom]. The results are shown in the Table 3.

Example 32

After 30 g of soluble starch, 7 g of polypeptone, 5 g of yeast extract and 5 g of potassium dihydrogenphosphate were dissolved in 1ℓ of distilled water, the pH of the solution was adjusted to 5.0 with 6N hydrochloric acid. After 10 ml of the above liquid medium was put in a test tube of 24 mm diameter and pluged with cotton, it was sterilized at 120° C under high pressure for 15 minutes. A loopful of cell of Arthrobacter globiformis IFO-12958 was inoculated to the medium and subjected to shaking culture at 30° C for 24 hours, which was used as seed culture. After 300 ml of a liquid medium having the same composition as above was put in a 2ℓ Sakaguchi's flask and sterilized in the same manner as above, 5 mℓ of the seed culture prepared as above was inoculated to the sterilized liquid medium and was subjected to shaking culture at 30° C for 30 hours. After that, the resulting culture was centrifuged to harvest 5 g (wet weight) of cells. The cells were suspended in 20 ml of 0.3M NaOH-$Na_2CO_3$ buffer solution (pH 10) and then 0.1 g of

ethyl (±)-cis-2,2-dimethyl-3-(2-chloro-3,3,3-trifluoropropenyl)cyclopropane carboxylate was added thereto. The mixture was allowed to react with stirring at 50°C for 118 hours. Two ml of 35% hydrochloric acid was added to the reaction solution and the resulting mixture was applied to extraction with 50 ml of methyl isobutyl ketone. The extract was then analyzed by gas chromatography (column: Shinchrom F-51 (5%) + $H_3PO_4$(1%),2.6 m, 185°C) and the yield of cis-2,2-dimethyl-3-(2-chloro-3,3,3-trifluoropropenyl)cyclopropane carboxylic acid was calculated from the ratio of the peak area of this compound to that of ethyl cis-2,2-dimethyl-3(2-chloro-3,3,3-trifluoropropenyl)cyclopropane carboxylate. All of the starting ethyl cis-2,2-dimethyl-3(2-chloro-3,3,3-trifluoropropenyl)cyclopropane carboxylate were recovered in the form as it was excluding that which had been converted to cis-2,2-dimethyl-3(2-chloro-3,3,3-trifluoropropenyl)cyclopropane carboxylic acid. To the extract was added IN sodium hydroxide solution to produce a sodium salt of cis-2,2-dimethyl-3(2-chloro-3,3,3-trifluoropropenyl)cyclopropane carboxylic acid and then this salt was extracted into an aqueous layer. The pH of the aqueous layer was adjusted with HCl solution to 2 or lower to liberate cis-2,2-dimethyl-3(2-chloro-3,3,3-trifluoropropenyl)cyclopropane carboxylic acid and this compound was extracted with methyl isobutyl ketone. The extract was concentrated to obtain almost pure cis-2,2-dimethyl-3-(2-chloro-3,3,3-trifluoropropenyl)cyclopropane carboxylic acid.

After 5 mg of the above cis-2,2-dimethyl-3(2-chloro-3,3,3-trifluoropropenyl)cyclopropane carboxylic acid was dissolved in 1 ml of toluene, equal molar amount each of thionyl chloride, pyridine and 3,5-dichloroaniline were added and the mixture was allowed to react to produce an anilide, which was subjected to analysis for optical isomers by high performance liquid chromatography (column: SUMIPAX OA-2100, moving phase: n-hexane/dichloroethane = 17/3, flow rate:1.0 ml/minute). The results are shown in the following table. The hydrolysis rate shown in the table represents the molar ratio of the resulting cis-2,2-dimethyl-3(2-chloro-3,3,3-trifluoropropenyl)cyclopropane carboxylic acid to ethyl (±)-cis-2,2-dimethyl-3(2-chloro-3,3,3-trifluoropropenyl)cyclopropane carboxylate used as the starting material.

Table

| Hydrolysis rate (%)<br>(%) | Ratio of the isomers of the resulting cis-2,2-dimethyl-3(2-chloro-3,3,3-trifluoropropenyl)cyclopropane carboxylic acid.<br>(+)-isomer/(-)-isomer |
|---|---|
| 8.7 | 100/0 |

Example 33

After 10 g of soluble starch, 5 g of yeast extract, 5 g of polypeptone, 1 g of potassium dihydrogenphosphate and 0.2 g of magnesium sulfate heptahydrate were dissolved in 1ℓ of distilled water, the pH of the solution was adjusted to 9 with 10% sodium carbonate solution. After 10 ml of the thus prepared liquid medium was put in a test tube and sterilized in the same manner as in Example 32, a loopful of cells of Bacillus sp. DC-1 was inoculated into the sterilized liquid medium and was subjected to shaking culture at 30°C for 24 hours to prepare a seed culture. After 300 ml of a liquid medium which was the same composition as above was put in a Sakaguchi's flask of 2ℓ capacity and sterilized in the same manner as above, 8 ml of the seed culture obtained as above was inoculated into the sterilized liquid medium and was subjected to shaking culture at 30°C for 24 hours. After that, the resulting culture solution was centrifuged to harvest 2 g (wet weight) of cells, which were then suspended in 20 ml of 0.1 M phosphate buffer solution (pH 8.0). To the suspension was added 0.1 g of monochloroethyl (±)-cis-2,2-dimethyl-3(2-chloro-3,3,3-trifluoropropenyl)cyclopropane carboxylate and the mixture was allowed to react with stirring at 40°C for 120 hours. After that, the same operation as used in Example 32 was performed and the results shown in the following table were obtained.

Table

| Hydrolysis rate (%) | Ratio of the isomers of the resulting cis-2,2-dimethyl-3(2-chloro-3,3,3-trifluoropropenyl)cyclopropane carboxylic acid. (+)-isomer/(-)-isomer |
|---|---|
| 28.2 | 93.1/6.9 |

Example 34

After 2ℓ of a liquid medium with the same composition as that used in Example 32 was put in a small fermentor and strilized at 120°C under high pressure for 15 minutes, 100 ml of the seed culture of Arthrobacter globiformis IFO-12958 prepared in the same manner as in Example 32 was inoculated into the sterilized medium and was subjected to aeration stirring culture at 30°C for 24 hours. Following that, the resulting cultured solution was centrifuged to harvest 53 g (wet weight) of cells. After the above harvested cells were suspended in 200 ml of 0.1M $NaOH-Na_2CO_3$ buffer solution (pH 10), the cells were then ground using a French press (product of the American Amico Company) and the cell debris were removed by centrifugation to obtain a crude enzyme solution. The resulting crude enzyme solution was then subjected to ammonium sulfate fractionation to collect a 30-60% saturation fraction and this fraction was lyophilized to obtain 1.3 g of a crude enzyme powder. After 0.5 g of the above crude enzyme powder was dissolved in 20 ml of 0.3 $NaOH-Na_2CO_3$ buffer solution (pH 10), 0.1 g of monochloroethyl (±)-cis-2,2-dimethyl-3-(2-chloro-3,3,3-trifluoropropenyl)cyclopropane carboxylate was added to the solution and the resulting mixture was allowed to react with string at 50°C for 117 hours. Two ml of 35% hydrochloric acid was added to the reaction mixture and the mixture was subjected to extraction with 50ml of methyl isobutyl ketone. After that, the same operation as used in Example 32 was performed and the results shown in the following table were obtained.

| Hydrolysis rate (%) | Ratio of the isomers of the resulting cis-2,2-dimethyl-3(2-chloro-3,3,3-trifluoropropenyl)cyclopropane carboxylic acid (+) isomer/(-)-isomer |
|---|---|
| 24.4 | 99.0/1.0 |

Table 1

| example | microorganism cultured | | Cis-2,2-dimethyl-3-(2,2-dichlorovinyl)cyclopropane carboxylic acid | |
|---|---|---|---|---|
| | | | Yield (%) | (+)-isomer/(-)-isomer |
| 1 | <u>Rhodosporidium toruloides</u> | IFO-0559 | 29.6 | 100/0 |
| 2 | <u>Rhodosporidium toruloides</u> | IFO-0871 | 22.8 | 100/0 |
| 3 | <u>Rhodosporidium toruloides</u> | IFO-8766 | 16.1 | 100/0 |
| 4 | <u>Rhodotorula glutinis</u> | IFO-1501 | 15.3 | 95.9/4.1 |
| 5 | <u>Candida tropicalis</u> | IFO-0618 | 4.10 | 92.0/8.0 |
| 6 | <u>Hansenula anomala var. ciferii</u> | IFO-0994 | 8.00 | 91.0/9.0 |
| 7 | <u>Torulopsis candida</u> | IFO-0768 | 11.0 | 85.0/15.0 |
| 8 | <u>Arthrobacter citreus</u> | IFO-12957 | 6.50 | 92.0/8.0 |
| 9 | <u>Pseudomonas putida</u> | IFO-12996 | 6.48 | 89.1/10.9 |
| 10 | <u>Escherichia coli</u> | IFO-13168 | 2.41 | 93.5/6.5 |

| | | | | |
|---|---|---|---|---|
| 11 | *Bacillus licheniformis* | IFO-12195 | 4.14 | 90.0/10.0 |
| 12 | *Flavobacterium capsulatum* | IFO-12533 | 6.39 | 88.2/11.8 |
| 13 | *Chromobacterium chocolatum* | IFO-3758 | 0.84 | 96.7/3.3 |
| 14 | *Achromobacter lyticus* | ATCC-21456 | 2.16 | 95.4/4.6 |
| 15 | *Rhizomucor pusillus* | IFO-9856 | 2.84 | 95.2/4.8 |
| 16 | *Flammulina velutipes* | IFO-7046 | 1.56 | 91.1/8.9 |
| 17 | *Geotrichum candidum* | IFO-4597 | 6.28 | 88.3/11.7 |
| 18 | *Dipodascus uninucleatus* | ATCC-14626 | 3.22 | 85.4/14.6 |
| 19 | *Beauveria bassiana* | ATCC-26037 | 1.80 | 89.4/10.6 |
| 20 | *Metschinikowia pulcherrima* | IFO-0561 | 4.20 | 98.2/1.8 |
| 21 | *Kluyveromyces lactis* | IFO-1090 | 0.48 | 99.1/0.9 |
| 22 | *Frateuria aurantia* | IFO-3247 | 3.96 | 88.0/12.0 |
| 23 | Klebsiella pneumoniae | IFO-12059 | 3.96 | 79.1/20.9 |

Table 2

| example | microorganism cultured | | Cis-2,2-dimethyl-3-(2,2-dimethylvinyl)cyclopropane carboxylic acid | |
|---|---|---|---|---|
| | | | Yield (%) | (+)-isomer/(-)-isomer |
| 24 | Rhodosporidium toruloides | IFO-0559 | 25.4 | 100/0 |
| 25 | Rhodosporidium toruloides | IFO-0871 | 19.9 | 100/0 |
| 26 | Rhodosporidium toruloides | IFO-8766 | 17.8 | 100/0 |
| 27 | Rhodotorula glutinis | IFO-1501 | 13.3 | 97.3/2.7 |

Table 3

| example | microorganism cultured | | Cis-2,2-dimethyl-3-(2,2-dibromovinyl)cyclopropane carboxylic acid | |
|---|---|---|---|---|
| | | | Yield (%) | (+)-isomer/(-)-isomer |
| 28 | Rhodosporidium toruloides | IFO-0559 | 31.1 | 100/0 |
| 29 | Rhodosporidium toruloides | IFO-0871 | 18.4 | 100/0 |
| 30 | Rhodosporidium toruloides | IFO-8766 | 19.3 | 100/0 |
| 31 | Rhodotorula glutinis | IFO-1501 | 15.5 | 96.4/3.6 |

## Claims

1. A process for producing an optically active (+)-cis-cyclopropane carboxylic acid or salt thereof which comprises reacting a (±)-cis-cyclopropane carboxylic acid ester represented by the formula (II)

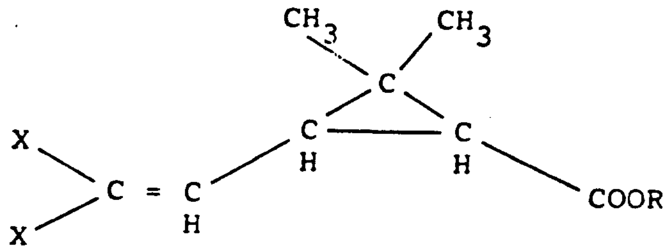

(II)

wherein each X, which may be the same or different represents chlorine, bromine, methyl or trifluoromethyl, and R represents a $C_1$-$C_4$ alkyl group or a $C_1$-$C_4$ alkyl group substituted by halogen atom, which formula does not indicate the configuration of the ester, with a microorganism selected from

| | |
|---|---|
| Rhodosporidium toruloides | IFO-0559 |
| Rhodosporidium toruloides | IFO-0871 |
| Rhodosporidium toruloides | IFO-8766 |
| Rhodotorula glutinis | IFO-1501 |
| Candida tropicalis | IFO-0618 |
| Hansenula anomala var. ciferii | IFO-0994 |
| Torulopsis candida | IFO-0768 |
| Arthrobacter citreus | IFO-12957 |
| Pseudomonas putida | IFO-12996 |
| Escherichia coli | IFO-13168 |
| Bacillus licheniformis | IFO-12195 |
| Flavobacterium capsulatum | IFO-12533 |
| Chromobacterium chocolatum | IFO-3758 |
| Achromobacter lyticus | ATTC-21456 |
| Rhizomucor pusillus | IFO-9856 |
| Flammulina velutipes | IFO-7046 |
| Geotrichum candidum | IFO-4597 |
| Dipodascus uninucleatus | ATTC-14626 |
| Beauveria bassiana | ATTC-26037 |
| Metschinikowia pulcherrima | IFO-0561 |
| Kluyveromyces lactis | IFO-1090 |
| Frateuria aurantia | IFO-3247 |
| Klebsiella pneumoniae | IFO-12059 and |
| Pediococcus acidilactici | IFO-3076 |

or an esterase produced by said microorganism to selectively and asymmetrically hydrolyze the ester of formula (II) to produce an optically active (+)-cis-cyclopropane carboxylic acid of formula (I) or salt thereof:

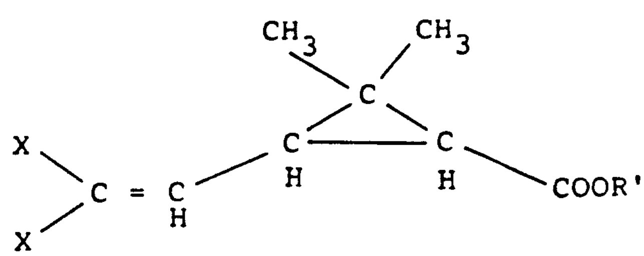

(I)

wherein X is as herein defined and R' represents hydrogen or a metal ion, which formula does not indicate the configuration of the acid, and (-)-cis-cyclopropane carboxylic acid ester of formula (II) and then recovering the optically active (+)-cis-cyclopropane carboxylic acid of formula (I) or salt thereof.

**2.** A process according to claim 1, wherein X represents chlorine, bromine or methyl and R represents a $C_1$-$C_4$ alkyl group.

**3.** A process for producing a (+)-cis-cyclopropane carboxylic acid or salt thereof represented by the formula (I-a)

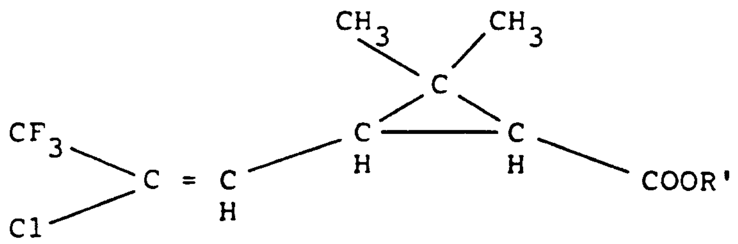

(I-a)

wherein R' represents hydrogen atom or metal ion, which formula does not indicate the configuration of the derivative or salt, which process comprises reacting a (±)-cis-cyclopropane carboxylic acid ester of the formula (II-a):

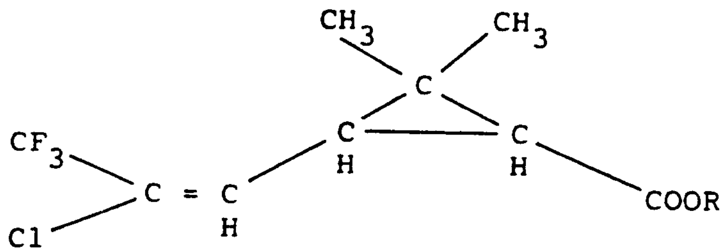

(II-a)

wherein R represents a $C_1$-$C_4$ alkyl group or $C_1$-$C_4$ alkyl group substituted by halogen atom, which formula does not indicate the configuration, with a microorganism which is Arthrobacter globiformis IFO-12958 or Bacillus sp. DC-1 (BP-1254) or an esterase produced by said microorganism.

## Patentansprüche

**1.** Verfahren zur Herstellung einer optisch aktiven (+)-cis-Cyclopropancarbonsäure oder eines Salzes davon, umfassend die Umsetzung eines (±)-cis-Cyclopropancarbonsäureesters der Formel (II)

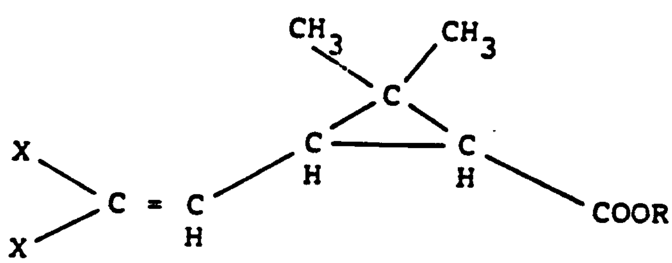

(II)

in der der Rest X, der gleich oder verschieden sein kann, ein Chloratom, Bromatom, eine Methyl- oder eine Trifluormethylgruppe bedeutet und R einen $C_1$-$C_4$-Alkylrest oder einen mit einem Halogenatom substituierten $C_1$-$C_4$-Alkylrest bedeutet, wobei die Formel nicht die Konfiguration des Esters angibt, mit einem Mikroorganismus ausgewählt aus

| | |
|---|---|
| Rhodosporidium toruloides | IFO-0559 |
| Rhodosporidium toruloides | IFO-0871 |
| Rhodosporidium toruloides | IFO-8766 |
| Rhodotorula glutinis | IFO-1501 |
| Candida tropicalis | IFO-0618 |
| Hansenula anomala var. ciferii | IFO-0994 |
| Torulopsis candida | IFO-0768 |
| Arthrobacter citreus | IFO-12957 |

| | |
|---|---|
| Pseudomonas putida | IFO-12996 |
| Escherichia coli | IFO-13168 |
| Bacillus licheniformis | IFO-12195 |
| Flavobacterium capsulatum | IFO-12533 |
| Chromobacterium chocolatum | IFO-3758 |
| Achromobacter lyticus | ATTC-21456 |
| Rhizomucor pusillus | IFO-9856 |
| Flammulina veluptipes | IFO-7046 |
| Geotrichum candidum | IFO-4597 |
| Dipodascus uninucleatus | ATTC-14626 |
| Beauveria bassiana | ATTC-26037 |
| Metschinikowia pulcherrima | IFO-0561 |
| Kluyveromyces lactis | IFO-1090 |
| Frateuria aurantia | IFO-3247 |
| Klebsiella pneumoniae | IFO-12059 |
| Pediococcus acidilactici | IFO-3076 |

oder einer von diesen Mikroorganismen erzeugten Esterase für die selektive und asymmetrische Hydrolyse des Esters der Formel (II) zur Herstellung einer optisch aktiven (+)-cis-Cyclopropancarbonsäure der Formel (I) oder eines Salzes davon:

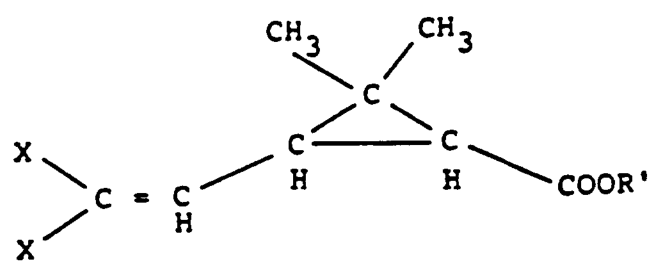

(I)

in der X wie vorstehend definiert ist und R' ein Wasserstoffatom oder ein Metallion bedeutet, wobei die Formel nicht die Konfiguration der Säure angibt, und eines (-)-cis-Cyclopropancarbonsäureesters der Formel (II), und anschließende Gewinnung der optisch aktiven (+)-cis-Cyclopropancarbonsäure der Formel (I) oder eines Salzes davon.

**2.** Verfahren nach Anspruch 1, wobei X ein Chlor- oder Bromatom oder eine Methylgruppe bedeutet und R einen $C_1$-$C_4$-Alkylrest darstellt.

**3.** Verfahren zur Herstellung einer (+)-cis-Cyclopropancarbonsäure oder eines Salzes davon mit der Formel (I-a)

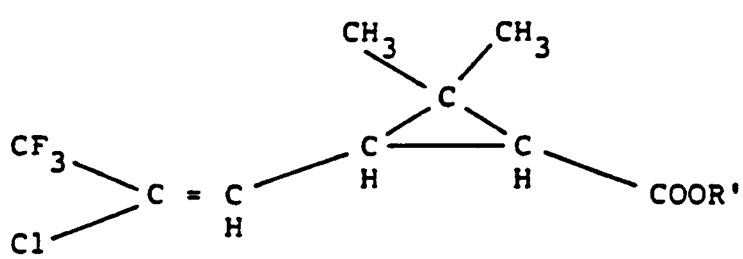

(I-a)

in der R' ein Wasserstoffatom oder ein Metallion bedeutet, wobei die Formel nicht die Konfiguration des Derivates oder Salzes angibt, umfassend, die Umsetzung eines (±)-cis-Cyclopropancarbonsäureesters der Formel (II-a):

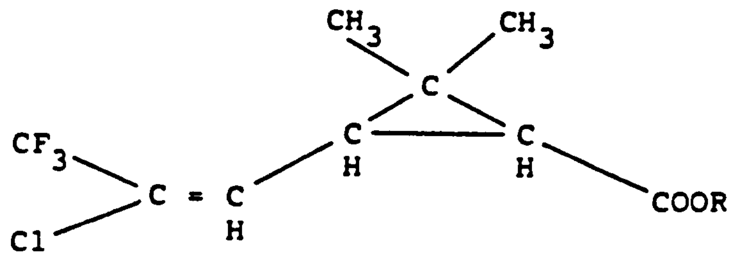

(II-a)

in der R einen $C_{1-4}$-Alkylrest oder einen mit einem Halogenatom substituierten $C_1$-$C_4$-Alkylrest bedeutet, wobei die Formel nicht die Konfiguration angibt, mit einem Mikroorganismus, nämlich Arthrobacter globiformis IFO-12958 oder Bacillus sp. DC-1 (BP-1254) oder einer von diesen Mikroorganismen erzeugten Esterase.

**Revendications**

1. Procédé de préparation d'un acide cyclopropane carboxylique (+)-cis optiquement actif ou de l'un de ses sels, qui consiste à mettre un ester d'acide cyclopropane carboxylique (±-cis) de formule (II)

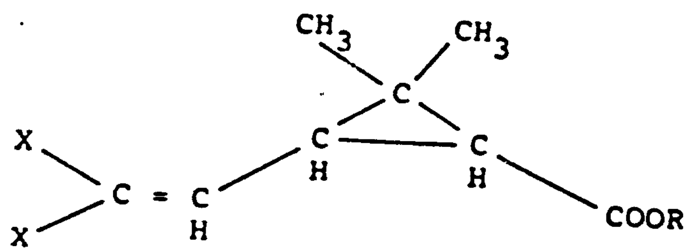

(II)

dans laquelle chaque X, qui peut être identique ou différent, représente le chlore, le brome, un méthyle ou un trifluorométhyle, et R représente un groupe alcoyle en $C_1$ à $C_4$ ou un groupe alcoyle en $C_1$ à $C_4$ substitué par un atome d'halogène, la formule n'indiquant pas la configuration de l'ester, à réagir sur un microorganisme choisi parmi

| | |
|---|---|
| Rhodoporidium toruloides | IFO-0559 |
| Rhodoporidium toruloides | IFO-0871 |
| Rhodoporidium toruloides | IFO-8766 |
| Rhodotorula glutinis | IFO-1501 |
| Candida tropicalis | IFO-0618 |
| Hansenula anomala var. ciferii | IFO-0994 |
| Torulopsis candida | IFO-0768 |
| Arthrobacter citreus | IFO-12957 |
| Pseudomonas putida | IFO-12996 |
| Escherichia coli | IFO-13168 |
| Bacillus licheniformis | IFO-12195 |
| Flavobacterium capsulatum | IFO-12533 |
| Chromobacterium chocolatum | IFO-3758 |
| Achromobacter lyticus | ATTC-21456 |
| Rhizomucor pusillus | IFO-9856 |
| Flammulina velutipes | IFO-7046 |
| Geotrichum candidum | IFO-4597 |
| Dipodascus uninucleatus | ATTC-14626 |
| Beauveria bassiana | ATTC-26037 |
| Metschinikowia pulcherrima | IFO-0561 |
| Kluyveromyces lactis | IFO-1090 |
| Frateuria aurantia | IFO-3247 |
| Klebsiella pneumoniae | IFO-12059 et |

Pediococcus acidilactici IFO-3076

ou sur une estérase produite par ce microorganisme pour hydrolyser sélectivement et asymétriquement l'ester de formule (II) en un acide cyclopropane carboxylique (+)-cis optiquement actif de formule (I) ou en son sel

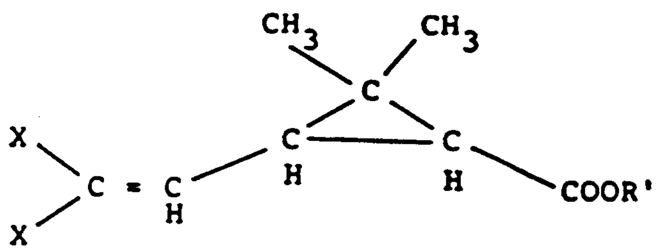

(I)

dans laquelle X est tel que défini ci-dessus et R' représente l'hydrogène ou un ion métallique, cette formule n'indiquant pas la configuration de l'acide, et un ester d'acide cyclopropane carboxylique (-)-cis de formule (II), puis à recueillir l'acide cyclopropane carboxylique (+)-cis optiquement actif de formule (I) ou son sel.

**2.** Procédé suivant la revendication 1, dans lequel X est le chlore, le brome ou le méthyle et R est un groupe alcoyle en $C_1$ à $C_4$.

**3.** Procédé de préparation d'un acide cyclopropane carboxylique (+)-cis ou de son sel de formule (I-a)

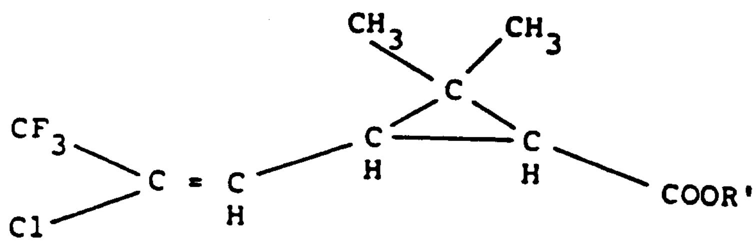

(I-a)

dans laquelle R' est un atome d'hydrogène ou un ion métallique, cette formule n'indiquant pas la configuration du dérivé ou du sel, qui consiste à mettre un ester d'acide cyclopropane carboxyllique (±)-cis de formule (II-a) :

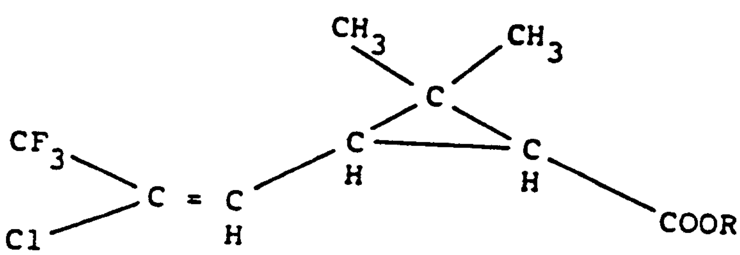

(II-a)

dans laquelle R est un groupe alcoyle en $C_1$ à $C_4$ ou un groupe alcoyle en $C_1$ à $C_4$ substitué par un atome d'halogène, cette formule n'indiquant pas la configuration, à réagir sur un microorganisme qui est Arthrobacter globiformlis IFO-12958 ou Bacillus sp. DC-1 (BP-1254) ou sur une estérase produite par ce microorganisme.